# EUROPÄISCHE PATENTANMELDUNG

(11) **EP 4 279 484 A1**
(43) Veröffentlichungstag der Anmeldung: **22.11.2023**
(21) Anmeldenummer: 22173701.8
(22) Anmeldetag: 17.05.2022
(51) Int. Cl.: C07D 211/74

(54) **VERBESSERTES VERFAHREN ZUR HERSTELLUNG VON TRIACETONAMIN**

(71) Anmelder: Sabo GmbH, 45772 Marl (DE)
(72) Erfinder: NEUMANN, Manfred, 45770 Marl (DE); HEIDELBERG, Julia, 48143 Münster (DE); MINKE, Katharina, 45128 Essen (DE); GÄRTNER, Felix, 45721 Haltern am See (DE)
(74) Vertreter: Herzog IP Patentanwalts GmbH

(57) **Zusammenfassung**

Die vorliegende Erfindung betrifft ein Verfahren zur Herstellung von Triacetonamin. Das Verfahren wird insbesondere kontinuierlich durchgeführt. Dabei wird ein Strom **S₁** umfassend Aceton und Ammoniak zwischen zwei Oberflächen **O₁** und **O₂** derselben Membran **M₁** oder zweier unterschiedlicher Membranen **M₁** und **M₂** geleitet, die in Fließrichtung des Stromes **S₁** um eine Achse **A** aufgewickelt ist/sind. Die Membran bzw. die Membranen umfassen eine feste fluorierte Sulfonsäure **K₁**, die die Umsetzung von Aceton und Ammoniak zu Triacetonamin katalysiert.

Das erfindungsgemäße Verfahren zeichnet sich dadurch aus, dass das erhaltene Triacetonamin ein vorteilhafteres Nebenproduktspektrum aufweist. Des Weiteren wurde überraschend festgestellt, dass die Membran durch die erfindungsgemäße Anordnung weniger Fluorid in das gewünschte Produkt abgibt. Das erfindungsgemäße Verfahren ermöglicht damit aufgrund der gezielten Anströmung der Katalysatoroberfläche eine verbesserte Katalysatorausnutzung und somit eine, auf das Gewicht des Katalysators bezogene, höhere Aktivität.

## Beschreibung

Die vorliegende Erfindung betrifft ein Verfahren zur Herstellung von Triacetonamin. Das Verfahren wird insbesondere kontinuierlich durchgeführt. Dabei wird ein Strom **S₁** umfassend Aceton und Ammoniak zwischen zwei Oberflächen **O₁** und **O₂** derselben Membran **M₁** oder zweier unterschiedlicher Membranen **M₁** und **M₂** geleitet, die in Fließrichtung des Stromes **S₁** um eine Achse A aufgewickelt ist/sind. Die Membran bzw. die Membranen umfassen eine feste fluorierte Sulfonsäure **K₁**, die die Umsetzung von Aceton und Ammoniak zu Triacetonamin katalysiert.

Das erfindungsgemäße Verfahren zeichnet sich dadurch aus, dass das erhaltene Triacetonamin ein vorteilhafteres Nebenproduktspektrum aufweist. Des Weiteren wurde überraschend festgestellt, dass die Membran durch die erfindungsgemäße Anordnung weniger Fluorid in das gewünschte Produkt abgibt. Das erfindungsgemäße Verfahren ermöglicht damit aufgrund der gezielten Anströmung der Katalysatoroberfläche eine verbesserte Katalysatorausnutzung und somit eine, auf das Gewicht des Katalysators bezogene, höhere Aktivität.

### Hintergrund der Erfindung

Triacetonamin (2,2,6,6-Tetramethyl-4-piperidinon; CAS-Nummer: 826-36-8; im Folgenden "TAA") ist ein wichtiges chemisches Intermediat, welches zur Synthese zahlreicher Folgeprodukte wie beispielsweise Lichtstabilisatoren (*hindered amine light stabilizers;* [HALS]), Oxidationsmitteln und Polymerisationsmoderatoren (z. B. Nitroxyl-Radikale) eingesetzt wird.

Die Herstellung von Triacetonamin aus Aceton und Ammoniak ist in Form verschiedener Verfahren dokumentiert. Dabei gliedern sich die Herstellungsverfahren in die direkte (einschrittige) Synthese von TAA aus den Edukten, beispielsweise beschrieben in DE 24 29 937 A1, US 4,536,581 A, JPS54-88275 A oder in Zeitschrift für Naturforschung 1976, 328 - 337 und 338 - 345, sowie die indirekte (zweistufige) Synthese über Acetonin (2,2,4,4,6-Pentamethyl-1,2,5,6-tetrahydro-pyrimidin), beispielsweise beschrieben in DE 24 29 935 A1 oder DE 24 29 936 A1, oder über Phoron (2,6-Dimethyl-2,5-heptadien-4-on), z. B. beschrieben in DE 2 352 127 A1. Bei der zweistufigen TAA-Synthese über Acetonin wird dies zunächst ausgehend von Aceton und Ammoniak gebildet, und reagiert dann in einem anschließenden Schritt unter Abspaltung von einem Äquivalent Ammoniak weiter zu TAA. Im Falle des Syntheseverfahrens über Acetonin werden jedoch immer beide Spezies (TAA und Acetonin) gleichzeitig gebildet, die Acetoninbildung ist kinetisch allerdings stark gegenüber der TAA-Bildung bevorzugt. In der "einstufigen" TAA-Synthese wird Acetonin lediglich nicht isoliert.

Die Herstellung von TAA ist grundsätzlich sowohl homogen katalysiert (meist durch Ammoniumsalze) als auch heterogen katalysiert (z. B. an sauren lonentauschern) möglich.

Die meisten Schriften aus dem Stand der Technik beziehen sich auf homogen katalysierte Reaktionen. Am häufigsten werden dabei Calciumchlorid (z.B. in Chemical Industries 2003, 89, 559-564; Zeitschrift für Naturforschung 1976, 328 - 337 und 338 - 345), Ammoniumchlorid (z. B. in xs; JP 2001-31651 A; JPH4-154762 A) und Hydrazinderivate (z. B. in JPS54-88275 A, JPS54-112873 A) genannt. Bei Einsatz dieser Katalysatoren treten allerdings Probleme auf. So hat etwa der Einsatz von Calciumchlorid den Nachteil, dass eine sehr langsame Reaktion erfolgt. Im Falle des Ammoniumchlorids ist die Reaktionsgeschwindigkeit höher, das eingesetzte Chlorid zeigt allerdings eine hohe Korrosivität gegenüber Stahl. Hydrazinderivate zeigen demgegenüber eine sehr hohe Toxizität.

Demgegenüber wurden auch Reaktionen an heterogenen Katalysatoren beschrieben, wie zum Beispiel in der DE 28 07 172 A1 und der CN 103224465 A.

Im Allgemeinen wird TAA in einer Matrix hergestellt, bei der das Aceton in großem Überschuss vorliegt und sowohl als Reaktionspartner als auch als Lösungsmittel dient. Daher ergibt sich am Ende der Reaktion ein Rohprodukt, welches neben TAA einen großen Anteil an Aceton, nicht umgesetztem Ammoniak, durch die Kondensation gebildeten Wassers und bei homogenkatalytischen Verfahren den Katalysator enthält. Darüber hinaus sind weitere Nebenkomponenten, wie z. B. acyclische Kondensationsprodukte (z. B. Diacetonalkohol, Diacetonamin, Mesityloxid, Phoron etc.), cyclische Kondensationsprodukte [z. B. Acetonin, 2,2,4,6-Tetramethyl-2,3-dihydropyridin (im Folgenden "TMDH-Pyridin")] oder höhermolekulare Kondensationsprodukte ("Hochsieder") enthalten.

Einige acyclische Additions- und Kondensationsprodukte (z. B. Diacetonalkohol [4-Hydroxy-4-methylpentan-2-on], Diacetonamin [4-Amino-4-methylpentan-2-on], Mesityloxid [4-Methylpent-3-en-2-on], Phoron etc.) können ihrerseits anstatt Aceton als Edukte mit Ammoniak zu TAA umgesetzt werden, was beispielsweise in Verfahren mit einem prozessinternen Recyclingstrom ausgenutzt wird (DE 28 07 172 A1).

Als heterogener Katalysator kommen feste, fluorierte Polysulfonsäuren und andere fluorierte Polymer mit kovalent gebundenen protischen Säuregruppen in Frage, die zu diesem Zweck beispielsweise in der EP 3 663 284 A1 und der EP 3 907 217 A1 beschrieben sind.

Diese fluorierten Polymere weisen beim Einsatz als heterogene Katalysatoren bei der TAA-Synthese allerdings den Nachteil der Auslaugung von Fluorid auf ("*leaching*", *"bleeding").* Somit besteht das Problem, dass bei der TAA-Synthese ein Rohprodukt erhalten wird, welches Fluoridionen aufweist. Dies ist unerwünscht, da es die Reinheit des erhaltenen TAA beeinträchtigt. Somit bestand ein Bedarf nach Verfahren zur Herstellung von TAA aus Aceton und Ammoniak, welches durch solche fluorierten Polymeren katalysiert wird, bei denen das Problem des Auswaschens von Fluoridionen vermindert ist.

Des Weiteren besteht das Bedürfnis, ein Verfahren zur Herstellung von TAA bereitzustellen, welches eine besseren Katalysatorausnutzung ermöglicht und zu einem TAA-Rohprodukt bzw. Reaktoraustrag mit einem vorteilhafteren Nebenproduktspektrum führt. "Vorteilhafteres Nebenproduktspektrum" bezieht sich damit insbesondere auf einen möglichst hohen Anteil an Nebenprodukten, die wieder als Edukte eingesetzt werden können (z.B. Mesityloxid) und/oder ein möglichst geringer Anteil an Nebenprodukten (z.B. TMDH-Pyridin), die nicht wieder als Edukte eingesetzt werden können.

Die Aufgabe der vorliegenden Erfindung bestand demnach darin, ein Verfahren zur Herstellung von TAA zur Verfügung zu stellen, welches die vorgenannten Probleme löst.

Insbesondere bestand die Aufgabe der vorliegenden Erfindung darin, ein Verfahren zur Herstellung von TAA aus Aceton und Ammoniak zur Verfügung zu stellen, welches unter Katalyse von auf fluorierten Polymeren kovalent gebundenen Säuregruppen, insbesondere Sulfonsäuregruppen, verläuft und eine verringerte Tendenz des Auswaschens von Fluoridionen aufweist.

### Kurzbeschreibung der Erfindung

Es wurde nun überraschend ein Verfahren gefunden, welches die erfindungsgemäße Aufgabe löst.

Das erfindungsgemäße Verfahren ist eines zur kontinuierlichen Herstellung von Triacetonamin,
wobei ein Strom **S₁** <1> umfassend Aceton und Ammoniak zwischen zwei Oberflächen **O₁** <11> und **O₂** <12> geleitet wird,
   (i) wobei die Oberflächen **O₁** <11> und **O₂** <12> Oberflächen einer um eine Achse **A** <3> gewickelten Membran **M₁** <4> sind, oder
   (ii) wobei die Oberfläche **O₁** <11> die Oberfläche einer Membran **M₁** <4> ist und die Oberfläche **O₂** <12> die Oberfläche einer von der Membran **M₁** <4> unterschiedlichen Membran **M₂** <5> ist, wobei **M₁** <4> und **M₂** <5> um dieselbe Achse **A** <3> gewickelt sind,
wobei die Achse **A** <3> parallel zur Fließrichtung des Stromes **S₁** <1> angeordnet ist,
wobei die Membranen **M₁** <4> und **M₂** <5> jeweils unabhängig voneinander ein fluoriertes Polymer **K₁** aufweisend kovalent gebundene Gruppen protischer Säuren umfassen,
insbesondere wobei die Membranen **M₁** <4> und **M₂** <5> jeweils unabhängig voneinander ein fluoriertes Polymer **K₁** aufweisend kovalent gebundene Gruppen ausgewählt aus Sulfonsäuregruppen, Carbonsäuregruppen, Gruppen der Strukturformel -P(=O)(OH)₂, Gruppen der Strukturformel -PH(=O)OH umfassen, und bevorzugt Sulfonsäuregruppen umfassen ,
und die Umsetzung von Aceton und Ammoniak zu Triacetonamin an **O₁** <11> und/oder **O₂** <12> durch **K₁** katalysiert wird,
wodurch ein Strom **S₂** <2> erhalten wird, der einen gegenüber **S₁** <1> erhöhten Gehalt an Triacetonamin umfasst.

### Abbildungen

Abbildung 1 zeigt eine Ausführungsform des erfindungsgemäßen Verfahrens mit einem Wickelmodul **W** <8>, in welches nur eine Membran **M₁** <4> schneckenhausförmig eingewickelt ist.

Abbildung 2 zeigt eine Ausführungsform des erfindungsgemäßen Verfahrens mit einem Wickelmodul **W** <8>, in welches mehrere Membranen, darunter auch die Membranen **M₁** <4> und **M₂** <5>, eingewickelt sind.

### Detaillierte Beschreibung der Erfindung

Das erfindungsgemäße Verfahren ist demnach ein, bevorzugt kontinuierliches, Verfahren zur Herstellung von Triacetonamin.

Dabei wird ein Strom **S₁** umfassend Aceton und Ammoniak zwischen zwei Oberflächen **O₁** und **O₂** geleitet.

Die Oberflächen **O₁** und **O₂** sind in einer bevorzugten erfindungsgemäßen Ausführungsform (i) die Oberflächen einer Membran **M₁**, die um eine Achse A gewickelt sind.

In einer alternativen erfindungsgemäßen Ausführungsform (ii) ist die Oberfläche **O₁** die Oberfläche einer Membran **M₁**, und die Oberfläche **O₂** ist die Oberfläche einer von der Membran **M₁** unterschiedlichen Membran **M₂**, wobei **M₁** und **M₂** um dieselbe Achse **A** gewickelt sind.

Das erfindungsgemäße Verfahren wird dabei bevorzugt in einem Wickelmodul **W** durchgeführt, in dem die Membran **M₁** bzw. die Membranen **M₁** und **M₂** in einer Außenwand **R** um eine Achse **A** gewickelt sind.

Solche Wickelmodule **W** sind dem Fachmann bekannt. Zum Beispiel sind dem Fachmann Wickelmodule **W**, die nach demselben Prinzip funktionieren, beispielsweise aus der Lebensmittelverarbeitung bekannt, zum Beispiel zur Filtration von Molke und Wein (siehe "prozesstechnik-online.de/dei0415423"; abrufbar unter https://prozesstechnik.industrie.de/food/verfahren-mechanisch-food/dicht-gepackt/, zuletzt abgerufen am 12. Mai 2022) oder zur Anwendung in der Katalyse in WO 2020/161263 A1.

In einem solche Wickelmodul **W**, das insbesondere für den Einsatz im erfindungsgemäßen Verfahren geeignet ist, ist die Membran **M₁** um eine Achse **A** gewickelt bzw. die Membranen **M₁** und **M₂** um dieselbe Achse **A** gewickelt.

Die Membranen **M₁** und **M₂** umfassen jeweils unabhängig voneinander ein fluoriertes Polymer **K₁** aufweisend kovalent gebundene Gruppen protischer Säuren.

Solche festen fluorierten Polymere bzw. Membranen sind dem Fachmann bekannt.

Es handelt sich dabei beispielsweise um Polymere wie fluoriertes Polyethylen, insbesondere Polyperfluorethylen, wobei diese Polymer kovalent gebunden funktionelle Gruppen ausgewählt aus -SO₃⁻, -COO⁻, -PO₃²⁻, -PO₂H⁻, bevorzugt -SO₃⁻ tragen. Solche Polymere sind beispielsweise in DE 10 2010 062 804 A1 und US 4,831,146 beschrieben.

Die an das fluorierte Polymer **K₁** kovalent gebundenen protischen Säuregruppen sind insbesondere aus der Gruppe bestehend aus Sulfonsäuregruppen -SO₃H (bzw. -SO₃⁻ im deprotonierten Zustand), Carbonsäuregruppen COOH (bzw. -COO⁻ im deprotonierten Zustand), Gruppen der Strukturformel -P(=O)(OH)₂ [bzw. -P(=O)(OH)O⁻ im deprotonierten Zustand; diese Gruppen sind von der Phosphorsäure bzw. phosphorigen Säure abgeleitet] oder -PH(=O)OH [bzw. -PH(=O)O⁻ im deprotonierten Zustand; diese Gruppen sind von der Phosphinsäure abgeleitet] ausgewählt.

Die an das fluorierte Polymer **K₁** kovalent gebundenen protischen Säuregruppen sind bevorzugt aus der Gruppe bestehend aus Sulfonsäuregruppen -SO₃H (bzw. -SO₃⁻ im deprotonierten Zustand), Carbonsäuregruppen COOH (bzw. -COO⁻ im deprotonierten Zustand) ausgewählt.

Besonders bevorzugt handelt es sich bei den an das fluorierte Polymer **K₁** kovalent gebundenen protischen Säuregruppen um Sulfonsäuregruppen -SO₃H (bzw. -SO₃⁻ im deprotonierten Zustand).

Solche fluorierten Polymere **K₁** mit kovalent gebundenen protischen Säuregruppen sind dem Fachmann beispielsweise aus der WO 2008/076327 A1, Absatz [058], US 2010/0044242 A1, Absatz [0042] oder der US 2016/0204459 A1 bekannt und unter dem Handelsnamen Nafion ^{®}, Aciplex ^{®} F, Flemion ^{®} erwerblich.

Besonders bevorzugt handelt es sich beim fluorierten Polymer **K₁** aufweisend kovalent gebundene Gruppen protischer Säuren um ein sulfoniertes Polyperfluorethylen, insbesondere Nafion ^{®} (CAS-Nummer: 31175-20-9).

Bevorzugt weist das fluorierte Polymer **K₁** aufweisend kovalent gebundene Gruppen protischer Säuren k₁ miteinander verknüpfte Wiederholungseinheiten der folgenden Struktur **P₁** auf, wobei k₁ eine ganze Zahl ≥ 2, insbesondere ≥ 4, bevorzugt ≥ 10, bevorzugter ≥ 50, noch bevorzugter ≥ 100, noch bevorzugter im Bereich 100 bis 10⁶, noch bevorzugter eine ganze Zahl im Bereich von 100 bis 10⁵, noch bevorzugter eine ganze Zahl im Bereich von 10² bis 10⁴ ist: 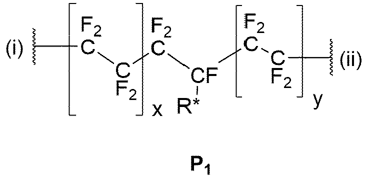
wobei x und y unabhängig voneinander jeweils eine ganze Zahl im Bereich 0 bis 10⁶, bevorzugter eine ganze Zahl von 1 bis 10⁵, noch bevorzugter eine ganze Zahl von 10 bis 10⁴, noch bevorzugter eine ganze Zahl von 10² bis 10³ sind,
wobei die Wiederholungseinheiten der chemischen Struktur **P₁** innerhalb des Polymers **K₁** gleich oder zumindest teilweise voneinander verschieden sind,
wobei die Wiederholungseinheiten der chemischen Struktur **P₁** innerhalb des Polymers **K₁** so miteinander verknüpft sind, dass die durch "(i)" gekennzeichnete Bindung einer bestimmten Wiederholungseinheit mit der durch "(ii)" gekennzeichneten Bindung der benachbarten Wiederholungseinheit verknüpft ist,
und wobei der Rest R* die folgende Struktur, wobei a = 0 oder 1, und wobei b = 0 oder 1, aufweist: 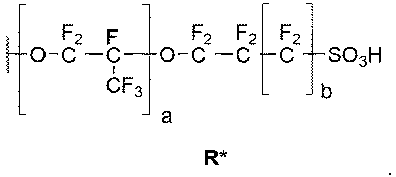
Bevorzugt ist a = 1 und b = 0.

Die Endgruppen des Polymers **K₁**, das heißt die Gruppen, die mit der Bindung (i) der ersten Widerholungseinheit und der Bindung (ii) der letzten Wiederholungseinheit verbunden sind, ergeben sich aus der Herstellung des Polymers **K₁**. Bevorzugt handelt es sich jeweils um -CF₃.

Bevorzugt weist das fluorierte Polymer **K₁** aufweisend kovalent gebundene Gruppen protischer Säuren k₂ miteinander verknüpfte Wiederholungseinheiten der folgenden Struktur **P_{Nafion}** auf, wobei k₂ eine ganze Zahl ≥ 2, insbesondere ≥ 4, bevorzugt ≥ 10, bevorzugter ≥ 50, noch bevorzugter ≥ 100, noch bevorzugter im Bereich 100 bis 10⁶, noch bevorzugter eine ganze Zahl im Bereich von 100 bis 10⁵, noch bevorzugter eine ganze Zahl im Bereich von 10² bis 10⁴ ist: 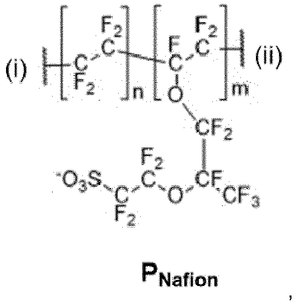
wobei n und m unabhängig voneinander jeweils eine ganze Zahl von 1 bis 10⁶, bevorzugter eine ganze Zahl von 10 bis 10⁵, noch bevorzugter eine ganze Zahl von 10² bis 10⁴ ist,
wobei die Wiederholungseinheiten der chemischen Struktur **P_{Nafion}** innerhalb des Polymers **K₁** gleich oder zumindest teilweise voneinander verschieden sind,
wobei die Wiederholungseinheiten der chemischen Struktur **P_{Nafion}** innerhalb des Polymers **K₁** so miteinander verknüpft sind, dass die durch "(i)" gekennzeichnete Bindung einer bestimmten Wiederholungseinheit mit der durch "(ii)" gekennzeichneten Bindung der benachbarten Wiederholungseinheit verknüpft ist.

Solche fluorierten Polymere **K₁**, welche kovalent gebundene Sulfonsäuregruppen aufweisen, sind als Membranen kommerziell erhältlich.

Die Membran **M₁** bzw. die Membranen **M₁** und **M₂** umfassend das fluorierte Polymer **K₁**, welches kovalent gebundene Gruppen protischer Säuren aufweist, können nach dem Fachmann bekannten Verfahren zu einem Wickelmodul **W** gewickelt werden.

In einer erfindungsgemäß bevorzugten Ausführungsform sind die Oberflächen **O₁** und **O₂** Oberflächen einer um eine Achse **A** gewickelten Membran **M₁**. Dies ist insbesondere dann der Fall, wenn nur eine Membran **M₁** im Wickelmodul **W** vorliegt und die vom Wickelmodul **W** umfasste Membran demnach einstückig ist.

In dieser erfindungsgemäß bevorzugten Ausführungsform ist die Membran **M₁** in Form eines Schneckenhauses (oder wie das Papier einer Küchenrolle) um die Achse **A** gewickelt. In anderen Worten ergibt der Querschnitt durch die gewickelte Membran **M₁** mit der Achse **A** als Normale zum Querschnitt in dieser Ausführungsform das Bild einer Spirale.

In einer alternativen erfindungsgemäßen Ausführungsform liegt die Membran, die im Verfahren eingesetzt wird, in mehreren Stücken, also nicht einstückig, vor.

In dieser alternativen Ausführungsform ist bevorzugt die Oberfläche **O₁** die Oberfläche einer Membran **M₁** und die Oberfläche **O₂** einer von der Membran **M₁** unterschiedlichen Membran **M₂**, wobei **M₁** und **M₂** um dieselbe Achse **A** gewickelt sind.

In dieser erfindungsgemäß bevorzugten Ausführungsform weisen die um die Achse **A** gewickelten Membranen, darunter auch die Membranen **M₁** und **M₂**, bevorzugt die Form eines Baumkuchens oder von Baumringen auf, d.h. der Querschnitt durch die um die Achse **A** gewickelten Membranen mit der Achse **A** als Normale zum Querschnitt ergibt das Bild konzentrischer (= ineinander liegender) Kreise oder konzentrischer Ellipsen.

In einer bevorzugten Ausführungsform des erfindungsgemäßen Verfahrens wird zwischen den beiden Oberflächen **O₁** und **O₂** mindestens ein Spacer **Sp** angeordnet. Der Spacer **Sp** hält die gegenüberliegenden Oberflächen der Membran **M₄** bzw. der Membranen **M₄** und **M₅** auseinander, so dass diese nicht miteinander verkleben und der Fluss des Stromes **S₁** in den Zwischenräumen zwischen den Oberflächen **O₁** und **O₂** nicht verhindert wird. Es versteht sich von selbst, dass der Spacer Sp aus einem Material ist und einen Aufbau aufweist, der den Fluss des Stromes **S₁** erlaubt. Insbesondere umfasst der Spacer **Sp** ein inertes Material. Noch bevorzugter besteht der Spacer aus einem inerten Material. Bevorzugt ist das inerte Material Kunststoff, bevorzugter Polypropylen.

Der Spacer **Sp** ist durchlässig für den Strom **S₁**. Es wird bevorzugt, dass der Spacer **Sp** porös ist, bevorzugter weist er die Struktur eines Netzes oder Vlieses auf.

Das erfindungsgemäße Verfahren wird bevorzugt in einem Wickelmodul **W** durchgeführt. Dazu wird die gewickelte Membran **M₁** bzw. werden die gewickelten Membranen, darunter **M₁** und **M₂**, in eine Außenwand **R**, der bevorzugt ein zylindrischer Behälter, bevorzugter ein kontinuierliches Strömungsrohr ist, eingeführt. Die Außenwand **R** ist bevorzugt aus einem inerten Material, welches unter den Bedingungen des erfindungsgemäßen Verfahrens stabil ist.

Die Fließrichtung des Stromes **S₁** im erfindungsgemäßen Verfahren ist parallel zur Achse **A**. Dies bedeutet, dass mindestens teilweise die Fließrichtung des Stromes **S₁** entlang der Zwischenräume zwischen den Oberflächen **O₁** und **O₂** verläuft.

Die Umsetzung von Aceton und Ammoniak zu Triacetonamin wird dann durch das fluorierte Polymer **K₁**, welches kovalent gebundene Gruppen protischer Säuren, insbesondere Sulfonsäuren aufweist, und bei dem es sich bevorzugt um Nafion handelt, katalysiert.

Die Umsetzung wie auch das gesamte erfinderische Verfahren wird bevorzugt kontinuierlich durchgeführt.

Dadurch wird ein Strom **S₂** erhalten, der einen gegenüber **S₁** erhöhten Gehalt an Triacetonamin umfasst. Der Strom **S₂** ist somit der Reaktoraustrag bzw. das Rohproduktgemisch der Reaktion. "Erhöhter Gehalt an Triacetonamin" bedeutet, dass die Konzentration von TAA im Strom **S₂** höher ist als im Eduktstrom **S₁**.

Der Strom **S₂** umfasst neben dem gewünschten Produkt TAA somit auch nicht reagiertes Aceton. Gegebenenfalls umfasst der Strom **S₂** auch nicht reagierten Ammoniak sowie Nebenprodukte. Als Nebenprodukt kommt insbesondere Mesityloxid in Frage. Mesityloxid ist das einfachste Kondensationsprodukt, das stets zumindest in geringer Menge bei der Umsetzung von Ammoniak und Aceton zu TAA durch Aldolkondensation zweier Moleküle Aceton gebildet wird. Auch höhermolekulare Kondensationsprodukte des Acetons mit sich oder Ammoniak entstehen bei der Umsetzung im erfindungsgemäßen Verfahren und sind deshalb insbesondere vom Strom **S₂** umfasst. Dies sind vor allem Nebenprodukte ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Isophoron.

Ein weiteres, unerwünschtes Nebenprodukt ist TMDH-Pyridin, welches im vorliegenden erfinderischen Verfahren in vorteilhaft geringer Menge gebildet wird.

Der Anteil des TAA, Aceton, Mesityloxid, Wasser und der genannten Nebenprodukte im Strom **S₂** ist nicht weiter beschränkt und ergibt sich aus der Stöchiometrie und den speziellen Reaktionsbedingungen. Der Anteil der jeweiligen Verbindung kann per GC bestimmt werden. Beispielsweise liegt nach der Reaktion ein Gemisch vor, in dem der Gehalt an Aceton bei 55 bis 60 Gew.-%, an Mesityloxid bei ≥ 11 Gew.-%, an TMDH-Pyridin bei < 4 Gew.-%, an der Summe aus Diacetonamin, Aicetonalkohol und Phoron bei 4 bis 6 Gew.-%, an TAA bei 14 bis 16 Gew.-%, und an höher als TAA siedenden Komponenten (zum Beispiel Iso-Phoron) bei 3 bis 4 Gew.-% liegt, und der Anteil von Wasser 7 Gew.-% ist.

Die "*volume space velocity"* für Ammoniak im kontinuierlichen Betrieb liegt im erfindungsgemäßen Verfahren insbesondere bei 0.01 bis 124.20 h⁻¹, bevorzugt 0.03 bis 5.25 h⁻¹, am bevorzugtesten bei 0.06 h⁻¹ (dies entspricht dem Volumen an Ammoniak, was pro Stunde und pro Volumen des Reaktors durch diesen Reaktor fließt, abgekürzt als "LHSV").

Die "*volume space velocity"* für Aceton im kontinuierlichen Betrieb liegt im erfindungsgemäßen Verfahren insbesondere bei 0.15 bis 1.33 h⁻¹, bevorzugt 0.66 bis 1.17 h⁻¹ (dies entspricht dem Volumen an Aceton, was pro Stunde und pro Volumen des Reaktors durch diesen Reaktor fließt).

Die Umsetzung im erfindungsgemäßen Verfahren kann in Gegenwart weiterer Lösungsmittel oder nur in Aceton, also ohne Zugabe weiterer Lösungsmittel, stattfinden. In den Fällen, in denen ein Lösungsmittel in Schritt (a) eingesetzt wird, können sämtliche Lösungsmittel eingesetzt werden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-*tert*-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, *tert*-Butanol; Ester, bevorzugt Methylacetat, Ethylacetat, Propylacetat, Butylacetat. Besonders bevorzugt findet die Umsetzung im erfindungsgemäßen Verfahren in Aceton, ohne Zugabe weiterer Lösungsmittel, statt.

Die Umsetzung im erfindungsgemäßen Verfahren wird vorzugsweise bei erhöhter Temperatur durchgeführt, insbesondere bei Temperaturen im Bereich von 20 °C bis 180°C, bevorzugt im Bereich von 40 °C bis 100 °C, bevorzugter im Bereich von 55 °C bis 90 °C, noch bevorzugter im Bereich von 60 °C bis 90 °C, am bevorzugtesten bei 75 °C.

Die Umsetzung im erfindungsgemäßen Verfahren wird insbesondere entweder bei Eigendruck der Komponenten oder bei erhöhtem Druck durchgeführt. So wird die Umsetzung bevorzugt bei einem Druck im Bereich von 1 bis 16 bar, bevorzugter bei einem Druck im Bereich von 1 bis 15 bar, noch bevorzugter bei einem Druck im Bereich von 7 bis 14 bar, noch viel mehr bevorzugter bei 12 bar durchgeführt.

Ammoniak wird im erfindungsgemäßen Verfahren bevorzugt als Reinstoff, d. h. als Gas zudosiert und liegt insbesondere während der Reaktion gelöst in Aceton bzw. gelöst im Reaktionsgemisch vor.

Aceton wird im erfindungsgemäßen Verfahren bevorzugt als Reinstoff zudosiert. Alternativ wird bevorzugt Aceton verwendet, was nach einer durchlaufenen Runde des erfindungsgemäßen Verfahrens abgetrennt werden und in einer neuen Runde rezykliert werden kann.

Daneben können im Strom **S₁** neben Aceton und Ammoniak Kondensationsprodukte des Acetons mit sich selbst oder Ammoniak vorliegen können. Diese können aus vorhergehenden Umsetzungsschritten von Ammoniak und Aceton zu TAA stammen.

Die Einsatzverhältnisse der Edukte im erfindungsgemäßen Verfahren können in weiten Bereichen gewählt werden, insbesondere wird Aceton im Überschuss zu Ammoniak eingesetzt. Bevorzugt beträgt das Molverhältnis von im erfindungsgemäßen Verfahren eingesetztem Aceton zu im erfindungsgemäßen Verfahren eingesetztem Ammoniak 3 : 1 bis 20 : 1, wobei ein Verhältnis von 6 : 1 bis 10 : 1 bevorzugt ist und ein Verhältnis von 7 : 1 besonders bevorzugt ist.

Die bevorzugt eingesetzte Menge an Polymer **K1** liegt bei 10 bis 20 Vol.-% bezogen auf die Gesamtmenge des im erfindungsgemäßen Verfahren eingesetzten Acetons.

Im Anschluss an die Umsetzung des erfindungsgemäßen Verfahrens kann dann TAA durch dem Fachmann bekannte Reinigungsmethoden aus dem Strom **S₂** abgetrennt werden. Bevorzugt werden zunächst Aceton und Mesityloxid abdestilliert, die dann als Teil des Stromes **S₁** recycliert werden können. Aus dem Destillationsrückstand kann dann TAA abdestilliert und das destillativ abgetrennte TAA aus der entsprechenden Fraktion bevorzugt auskristallisiert werden.

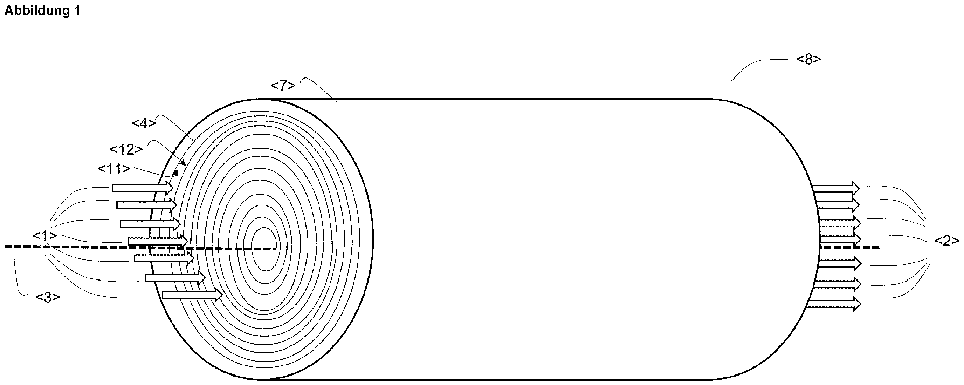

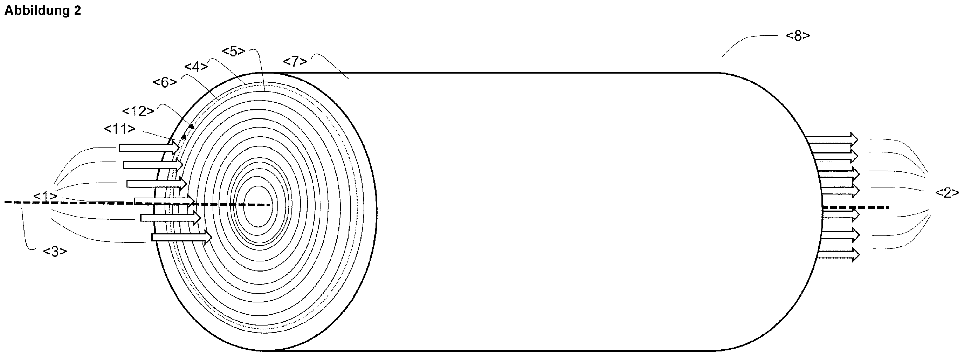

Die Abbildungen 1 und 2 zeigen zwei bevorzugte Ausführungsformen des erfindungsgemäßen Verfahrens.

Abbildung 1 zeigt eine bevorzugte Ausführungsform [gemäß Option (i) des Anspruchs 1] des erfindungsgemäßen Verfahrens anhand eines Wickelmoduls **W** <8>, in dem eine Membran **M₁** <4> schneckenhausförmig (bzw. wie das Papier einer Küchenrolle) um eine Achse **A** <3> gewickelt ist. Die Achse **A** <3> kann gedacht oder ein Befestigungselement sein, an dem die Membran **M₁** <4> zur einfacheren Wicklung befestigt ist.

Die so gewickelte Membran **M₁** <4> steckt in einer bevorzugt zylindrischen Außenwand **R** <7>. Die Gesamtheit der Membran **M₁** <4> und der Außenwand **R** <7> stellen das Wickelmodul **W** <8> in seiner grundlegendsten Ausführungsform dar. Die Membran **M₁** <4> umfasst eine perfluorierte Polysulfonsäure (Nafion ^{®}), die über die Oberfläche der Membran **M₁** <4>, z.B. ihre Oberflächen **O₁** <11> und **O₂** <12>, direkt kontaktierbar ist. Diese Bedingung ist in einer bevorzugten Ausführungsform der vorliegenden Erfindung erfüllt, wenn die Membran **M₁** <4> im Wesentlichen aus der perfluorierten Polysulfonsäure besteht.

Das Wickelmodul **W** <8> wird mit einem Strom **S₁** <1> umfassend Aceton und Ammoniak beschickt der sich gemäß den in Abbildung 1 gezeigten Pfeilen in den Zwischenräumen der Wicklungen der Membran **M₁** <4> verteilt und entlang den Zwischenräumen und parallel zur Achse durch das Wickelmodul **W** <8> fließt. Dabei werden Aceton und Ammoniak durch die Katalyse der fluorierten Sulfonsäure an den Oberflächen **O₁** <11> und **O₂** <12> zu TAA umgesetzt. Am Ende des Wickelmoduls **W** <8> wird somit ein Strom **S₂** <2> erhalten, der TAA und auch Nebenprodukte wie acyclische Kondensationsprodukte (z. B. Diacetonalkohol, Diacetonamin, Mesityloxid, Phoron), cyclische Kondensationsprodukte [z. B. Acetonin, TMDH-Pyridin] oder höhermolekulare Kondensationsprodukte enthält.

Abbildung 2 zeigt eine weitere bevorzugte Ausführungsform [gemäß Option (ii) des Anspruchs 1] des erfindungsgemäßen Verfahrens. Diese entspricht der in Abbildung 1 gezeigten Ausführungsform bis darauf, dass das Wickelmodul **W** <8> mehrere Membranen umfasst, die um dieselbe Achse **A** <3> gewickelt sind. Hervorgehoben sind dabei die beiden Membranen **M₁** <4> und **M₂** <5>. Die Oberfläche **O₁** <11> ist in dieser Ausführungsform eine Oberfläche der Membran **M₁** <4>, und Oberfläche **O₂** <12> ist eine Oberfläche der Membran **M₂** <5>. Abbildung 2 zeigt eine weitere bevorzugte Ausführungsform im Wickelmodul **W** <8>: zwischen den Membranen **M₁** <4> und **M₂** <5> ist ein Spacer **Sp** <6> angedeutet. Dieser Spacer **Sp** <6> verhindert das Verkleben von benachbarten Membranoberflächen und erhöht die Effizienz des Verfahrens. Entsprechende Spacer können zwischen weiteren und bevorzugt allen benachbarten Membranen im Wickelmodul **W** <8> angebracht sein. Da das Wickelmodul **W** <8> in der Ausführungsform gemäß Abbildung 2 mehrere Membranen umfasst, die um dieselbe Achse **A** <3> gewickelt sind, bilden die einzelnen Membranen konzentrische Kreise bzw. Ellipsen, die denselben Mittelpunkt haben bzw. ineinander liegen. Es erinnert daher in der Draufsicht an die Struktur der Lagen eines Baumkuchens.

### Beispiele

### Verqleichsbeispiel

Zwei in Reihe geschaltete zylindrische Reaktoren werden mit Nation^{®} SAC-13 (poröse Siliciumdioxidpartikel, auf dem Nation^{®} in einer Beladung von etwa 13 Gew.-% adsorbiert ist) so gefüllt, dass dieses Katalysatormaterial durch Siebe oben und unten begrenzt angeordnet ist. Das Schüttvolumen des Katalysators beträgt je ca. 800 mL im wasserfeuchten Zustand. Der Reaktor wird kontinuierlich mit 630 g/h Aceton und 25 g/h Ammoniak beschickt. Dabei wird eine Temperatur von 75 °C und ein Druck von 14 bar eingestellt.

Der Fluoridgehalt des Reaktoraustrag wird mit *inductive coupled plasma* (Elementaranalyse) bestimmt.

### Erfinderisches Beispiel

In zwei in Reihe geschaltete zylindrische Reaktoren wird jeweils ein Wickelmodul, auf welchem eine Nafion^{®}-Membran mit einer Spacerschicht (Polypropylen) entsprechend Abbildung 1 in einem Stahlreaktor gewickelt ist, eingehängt.

Das Wickelmodul wird kontinuierlich mit 630 g/h Aceton und 25 g/h Ammoniak beschickt. Dabei wird eine Temperatur von 75 °C und ein Druck von 14 bar eingestellt.

Der Fluoridgehalt des Reaktoraustrag wird per Elementaranalyse mit *inductively coupled plasma atomic emission spectroscopy* ("ICP-AES") bestimmt.

### Ergebnis

Der Fluoridgehalt im Reaktoraustrag ist bezogen auf die Masse des eingesetzten NAFIONs und bezogen auf das erhaltene TAA beim erfinderischen Vorgehen geringer.

## Patentansprüche

1. Verfahren zur Herstellung von Triacetonamin,
wobei ein Strom **S₁** <1> umfassend Aceton und Ammoniak zwischen zwei Oberflächen **O₁** <11> und **O₂** <12> geleitet wird,
(i) wobei die Oberflächen **O₁** <11> und **O₂** <12> Oberflächen einer um eine Achse **A** <3> gewickelten Membran **M₁** <4> sind, oder
(ii) wobei die Oberfläche **O₁** <11> die Oberfläche einer Membran **M₁** <4> ist und die Oberfläche **O₂** <12> die Oberfläche einer von der Membran **M₁** <4> unterschiedlichen Membran **M₂** <5> ist, wobei **M₁** <4> und **M₂** <5> um dieselbe Achse **A** <3> gewickelt sind,
wobei die Achse **A** <3> parallel zur Fließrichtung des Stromes **S₁** <1> angeordnet ist,
wobei die Membranen **M₁** <4> und **M₂** <5> jeweils unabhängig voneinander ein fluoriertes Polymer **K₁**, welches kovalent gebundene Gruppen protischer Säuren aufweist, umfassen,
und wobei die Umsetzung von Aceton und Ammoniak zu Triacetonamin an **O₁** <11> und/oder **O₂** <12> durch **K₁** katalysiert wird,
wodurch ein Strom **S₂** <2> erhalten wird, der einen gegenüber **S₁** <1> erhöhten Gehalt an Triacetonamin umfasst.

2. Verfahren nach Anspruch 1, wobei zwischen den Oberflächen **O₁** <11> und **O₂** <12> mindestens ein Spacer **Sp** <6> angeordnet ist.

3. Verfahren nach Anspruch 1 oder 2, wobei die Oberflächen **O₁** <11> und **O₂** <12> Oberflächen einer um eine Achse **A** <3> gewickelten Membran **M₁** <4> sind.

4. Verfahren nach Anspruch 3, wobei die Membran **M₁** <4> in Form eines Schneckenhauses um die Achse **A** <3> gewickelt ist.

5. Verfahren nach Anspruch 1 oder 2, wobei die Oberfläche **O₁** <11> die Oberfläche einer Membran **M₁** <4> ist und die Oberfläche **O₂** <12> die Oberfläche einer von der Membran **M₁** <4> unterschiedlichen Membran **M₂** <5> ist, wobei **M₁** <4> und **M₂** <5> um dieselbe Achse **A** <3> gewickelt sind.

6. Verfahren nach Anspruch 5, wobei der Querschnitt durch die um die Achse **A** <3> gewickelten Membranen mit der Achse **A** <3> als Normale zum Querschnitt das Bild konzentrischer Kreise oder konzentrischer Ellipsen ergibt.

7. Verfahren nach einem der Ansprüche 1 bis 6, welches in einem Wickelmodul **W** <8> durchgeführt wird.

8. Verfahren nach einem der Ansprüche 1 bis 7, wobei die protische Säuregruppe aus der Gruppe bestehend aus Sulfonsäuregruppen, Carbonsäuregruppen, Gruppen der Strukturformel -P(=O)(OH)₂, Gruppen der Strukturformel -PH(=O)OH ausgewählt ist.

9. Verfahren nach Anspruch 8, wobei die protische Säuregruppe eine Sulfonsäuregruppe ist.

10. Verfahren nach Anspruch 9, wobei das fluorierte Polymer **K₁** aufweisend kovalent gebundene Gruppen protischer Säuren k₁ miteinander verknüpfte Wiederholungseinheiten der folgenden Struktur **P₁** aufweist, wobei k₁ eine ganze Zahl ≥ 2 ist: 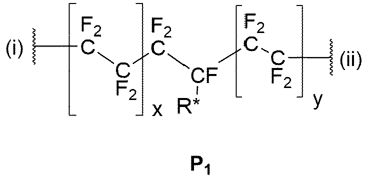
wobei x und y unabhängig voneinander jeweils eine ganze Zahl im Bereich 0 bis 10⁶ sind,
wobei die Wiederholungseinheiten der chemischen Struktur **P₁** innerhalb des Polymers **K₁** gleich oder zumindest teilweise voneinander verschieden sind,
wobei die Wiederholungseinheiten der chemischen Struktur **P₁** innerhalb des Polymers **K₁** so miteinander verknüpft sind, dass die durch "(i)" gekennzeichnete Bindung einer bestimmten Wiederholungseinheit mit der durch "(ii)" gekennzeichneten Bindung der benachbarten Wiederholungseinheit verknüpft ist,
und wobei der Rest R* die folgende Struktur aufweist: 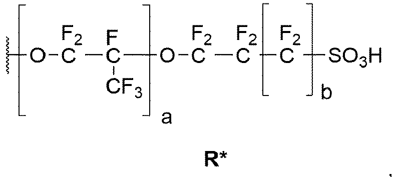
wobei a = 0 oder 1;
und wobei b = 0 oder 1.

11. Verfahren nach Anspruch 10, wobei a = 1 und b = 0.

12. Verfahren nach Anspruch 9, wobei das fluorierte Polymer **K₁** aufweisend kovalent gebundene Gruppen protischer Säuren k₂ miteinander verknüpfte Wiederholungseinheiten der folgenden Struktur **P_{Nafion}** aufweist, wobei k₂ eine ganze Zahl ≥ 2 ist: 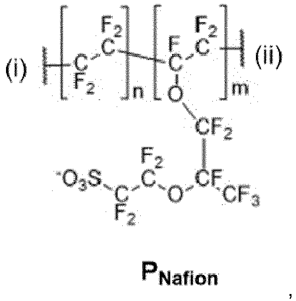
wobei n und m unabhängig voneinander jeweils eine ganze Zahl von 1 bis 10⁶ ist,
wobei die Wiederholungseinheiten der chemischen Struktur **P_{Nafion}** innerhalb des Polymers **K₁** gleich oder zumindest teilweise voneinander verschieden sind,
wobei die Wiederholungseinheiten der chemischen Struktur **P_{Nafion}** innerhalb des Polymers **K₁** so miteinander verknüpft sind, dass die durch "(i)" gekennzeichnete Bindung einer bestimmten Wiederholungseinheit mit der durch "(ii)" gekennzeichneten Bindung der benachbarten Wiederholungseinheit verknüpft ist.

13. Verfahren nach einem der Ansprüche 1 bis 12, welches bei einem Druck im Bereich von 1 bis 20 bar und einer Temperatur im Bereich von 20 °C bis 180 °C durchgeführt wird.
